# EUROPEAN PATENT SPECIFICATION

(11) **EP 0 783 900 B1**
(45) Date of publication and mention of the grant of the patent: **09.04.2003**
(21) Application number: 96850215.3
(22) Date of filing: 20.12.1996
(51) Int. Cl.: A61N 1/05

(54) **Implantable electrode cable device with at least one electrode contact means**
Implantierbares Elektrodenkabel mit mindestens einem Elektrodenkontakt
Câble d'électrode implantable comprenant au moins un contact d'électrode

(30) Priority: 28.12.1995 SE 9504675
(43) Date of publication of application: 16.07.1997
(73) Proprietor: St. Jude Medical AB, 175 84 Järfälla (SE)
(72) Inventor: Lindegren, Ulf, 122 35 Enskede (SE)
(74) Representative: Kalling, Sven Owe

(56) References cited:
- EP-A- 0 085 967
- US-A- 4 258 724
- US-A- 4 699 147
- US-A- 5 476 495

## Description

### Technical field

The present invention relates to an electrode cable device with a proximal end and a distal end and comprises one or a plurality of thin, insulated electrical conductors with a proximal end and a distal end, the conductor, or at least one of the conductors, having at least one implantable electrode contact means in electrical contact with the conductor and intended for permanent electrical contact with the wall of a cavity in a human body. The cable device is devised so the respective electrode contact means can be kept pressed, by means of pre-tensioning, against an area of the wall forming the cavity in the body. The body cavity can e.g. be an atrium or a ventricle of the heart.

### The prior art

An electrode cable device of the aforementioned kind is especially designed for introduction into a patient's heart and for anchoring the device's electrode contact means in the heart's ventricle and/or atrium. Such an electrode cable device is usually introduced into the heart via a vein, and the electrode cable means are usually anchored in the right ventricle or atrium of the heart. The proximal end of the electrode cable device is intended for connection to a heart stimulator which has been implanted into the body. The electrode cable device can then be used for carrying electrical impulses from the heart stimulator to the heart, via the implantable electrode contact means, and/or for sensing and registering heart signals.

US-A-4,522,212 describes an endocardiac electrode device comprising least three curved spring wires designed for introduction into the heart, each curved spring wire bearing an array of electrodes situated to form a characteristic geometric pattern across the ventricular contact area covered by the spring wires. The active electrode arrays achieve recognizable patterns when inside a heart ventricle and can be viewed with a fluoroscope. The electrodes are located in relation to each other and on the spring wires so the wires, with their electrodes, can easily be squeezed into a narrow spring wire bundle which fits inside a catheter during the catheter's introduction into the heart. The catheter can then be retracted a little, exposing electrodes on the curved spring wires which spring out into a balloon-like shape inside the heart ventricle. The curved spring wires consist of curved metal arches with external insulation on their entire length, except at the points at which electrodes are attached. When the electrode cable device has been implanted inside the heart, the spring wires retain their original pre-tensioning, thereby exerting constant pre-tensioning pressure on the electrodes.

US-A-4,699,147 describes a probe, equipped with a plurality of electrodes for intraventricular heart catheterization. The probe comprises a catheter with an open, proximal end, an open distal end and four elongate conductor devices, with a distal end section and a proximal end section, inside the catheter and projecting beyond the open, proximal end of the catheter. Each conductor device comprises a tubular sleeve, six insulated wire-like conductors inside the sleeve and a stiff but flexible, central core wire arranged inside the sleeve and covering most of the shell's length. A proximal contact means is mounted on each tubular shell. The distal end section of each conductor device carries six separate shell electrodes connected to each wire-like conductor. The section of each core wire at the distal end section of the conductor devices can be made to assume a desired configuration after the distal end sections of the conductor devices are moved from a retracted position inside the catheter to a position in which the distal end sections project outside the catheter, and the core wires can be made to assume a desired configuration in order to jointly form an elliptical wire sleeve. The catheter is designed for introduction into an artery or vein and placement of its distal end opening in a heart ventricle from said end opening the distal end sections of the conductor devices can be extended to form the elliptical wire shell and then rotated in stages while electrical potentials are measured and recorded at different points on the surface of endocardiac ventricular wall in contact with the shell electrodes. Even in this known type of electrode cable probe, the core wires retain their elliptical configuration from the time they are able to bulge out, as a result of their pre-tensioned elliptical shaping, and make contact with the ventricular wall of the heart. So the shell electrodes are kept permanently pressed against the ventricular wall of the heart as a result of the spring force residing in the core wires of the conductor devices and cannot be pulled out of same.

### Summary of the invention

A main object of the present invention is to achieve a new type of electrode device making it possible to position and anchor a plurality of small electrode means or tips at a corresponding number of sites in the ventricle and atrium of a heart and be applied, first with a given pressure (pre-tensioning), exerted by pressure-exerting stiffening means, against internal surface areas of the walls of the ventricle and atrium and then, after a given period of time when the electrode contacts means have become anchored/embedded in heart tissue, be firmly seated in the respective heart wall area without the continued exertion of any residual pressure by any elastic stiffening means.

The main idea of the invention is for the elastic means, which are used at an initial stage of electrode implantation to keep the electrode contact means pressed against the respective cavity wall, to successively degrade and dissolve after the requisite anchoring/ embedding of the electrode contact means has occurred, when the elastic means are no longer needed but could otherwise damage adjacent cavity wall if e.g. any of the elastic means were to fracture, an event which could cause lethal penetration of the cavity wall.

A related object of the invention is to select elastic means with properties enabling the body, through the action of body fluids, to break them down successively and cause them to disappear after a given, appropriate period of time.

The problems related to the aforementioned objects are solved according to the invention when an electrode cable device of the aforementioned kind displays the features set forth in the characterizing part of patent claim 1.

Preferred embodiments of the electrode cable device according to the invention can also display the features set forth in the dependent patent claims.

Thus, a primary distinctive feature of the electrode cable device is that conductors, or every conductor in the electrode cable device, is supported by an elongate, pre-shaped stiffening means, connected to and running along the conductor, made of an inherently elastic material which is resorbable *in vivo.* This stiffening means (which constitutes the above-discussed elastic means) is devised and arranged to impart an arched or helical shape to at least the section of the conductor on which its contact means are located. The electrode cable device also comprises a channel means, which extends longitudinally through the device, into which a stylet can be inserted to permit temporary, essentially linear straightening of the cable device and its conductor's arched or helical section.

The inherently elastic material, which is resorbable in *vivo*, shall, according to the invention, be made of a material which is biodegradable or soluble within an appropriate period of time after it comes into contact with blood. The resorbable or degradable material can preferably be selected from a group of proteins/amino acid polymers, poly(hydroxycarboxyl acids) and or carbohydrate polymers. The proteins/amino acid polymers group can contain gelatin, collagen, polyserine, polythreonine, polyphenylalanine or the like. The poly(hydroxycarboxyl acids) group can contain polylactides and/or polyglycolides. The carbohydrate polymers group can contain dextran, starch, hyaluronic acid, cellulose or the like.

Breakdown or degradation time for the material resorbable *in vivo* should be at least several hours but should generally be on the order of about 24 hours to three to four weeks or, in some instances, months.

In instances in which the electrode cable device comprises at least one, preferably two, pair of thin, insulated electrical conductors, each conductor in each pair of conductors can have an electrode means, serving as a microelectrode, arranged on a convex, arching section, which bulges out from the center line of the cable device, the conductors in each pair of conductors arching outwards in opposite directions.

With a cable device devised in this way, both conductors, equipped with stiffening means, in each conductor pair assume a convex, lens-shaped position-retaining means in the body cavity in which the conductor pair is located.

When the electrode cable device according to the invention is to be used for placing groups of electrode contact means in separate body cavities, such as the atrium and ventricle of a heart, a version is used in which two groups of electrode contact means are arranged in their respective contact areas by two longitudinally separate contact areas in the device. Here, each of the two contact areas is intended for one of two separate body cavities. A cable device devised in this way is set forth in patent claim 3. When such a cable device has four pairs of insulated electrical conductors, the electrode contact means can suitably be distributed among the contact areas according to patent claim 4.

When the electrode contact means are grouped for activity in different contact areas (in different body cavities), the electrical conductors plus attendant stiffening means form bundled cable strings within the cable section in which the conductors lack any electrode contact means, e.g. between the above-mentioned contact areas. One such embodiment is set forth in patent claim 5.

The pre-shaped stiffening means, which are connected to the conductors and extend along same, can be devised in different ways. In a particularly simple embodiment, the stiffening means consists of a tubular sleeve on the exterior of the conductor. Or the stiffening means can consist of a continuous band-shaped or wire-like stiffening means running along and attached to the conductor.

An additional alternative embodiment of the stiffening means for the conductor is set forth in patent claim 8.

In a cable device according to the latter claim, the channel means for a stylet can appropriately consist of a longitudinal channel inside the cable string sections, with a round cross-section, of the cable device.

The distal ends of the conductors in the anterior, first contact area are appropriately connected to the channel means on the distal end of the cable device.

The pre-shaped stiffening means running along the conductors, as described above, are made of an inherently elastic material which is resorbable *in vivo.* This material should be biodegradable and dissolve in the body within an appropriate period of time after being in contact with blood. The period of time must be such that the respective electrode contact means has time to become embedded in the tissue of the wall of the respective body cavity before the stiffening means degrades and dissolves through the action of blood. The degradation and removal by the blood of the elastic stiffening means after a given period of time accordingly completely removes said stiffening means along each conductor after this given period of time during which the electrode contact means have time to become embedded into the wall of the adjacent body cavity, thereby eliminating the need for stiffening, pressure-application means for the electrode contact means, thereby conveying a major advantage.

The risk of a future fatal fracture of the arched, electrode contact-carrying spring wires, as is present with the known types of electrode contact devices, e.g. according to the above-described U.S. patent documents, is accordingly avoided.

This risk obviously poses a life-menacing threat to the patient, since fatigue fracture of an implantable, elastic spring wire, left in the heart as an "electrode holder", could have fatal consequences if any part of the broken wire spring penetrated the wall of the ventricle or atrium, an event which should lead to death caused by internal bleeding.

### FIGURES

The invention will now be described and explained below in greater detail, referring to the enclosed drawings, which schematically depict an example of an electrode cable device according to the invention::
FIG. 1 shows a highly schematic lateral view of an electrode cable device, equipped with a plurality of electrode contact means, in its implantation state with electrode contact means bulging out to the side;
FIG. 2 shows an electrode cable device according to FIG. 1 with the electrode contact means radially "collapsed" by the insertion of an axial stylet into an axial channel means inside the electrode cable device, stretching same axially and longitudinally;
FIGS. 3 and 4 show cross-sections through the electrode device at section lines A-A and B-B in FIG. 1;
FIG. 5a and 5b show cross-sections at C-C and D-D in FIG. 1;
   FIG. 5c and 5d show cross-sections through alternative stiffening means, at C-C and D-D in FIG. 1; and
FIGS. 6 and 7 show cross-sections at E-E and F-F in FIG. 1.

### Preferred embodiment

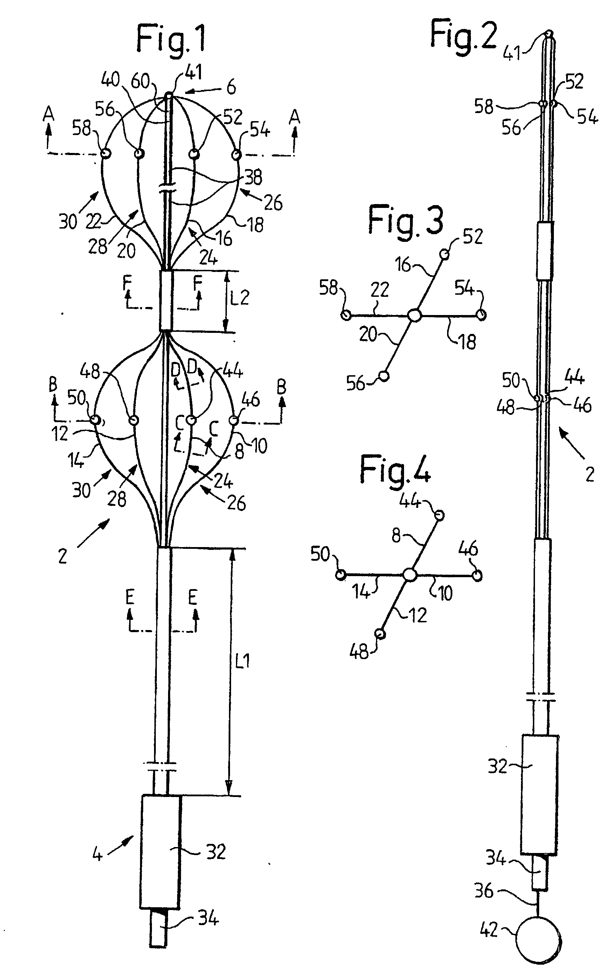

FIG. 1 schematically depicts an electrode cable device 2 according to the invention. The cable device has a proximal end 4 and a distal end 6 and comprises, in this instance, eight thin, flexible, insulated electrical conductors 8, 10, 12, 14, 16, 18, 20, 22, each with a proximal end and a distal end. Each conductor in the electrode cable device 2 is supported by an associated, elongate stiffening means 24, 26, 28, 30, connected to the conductor. Each such stiffening means 24-30 extends along at least part of the conductors. In the illustrated instance, the stiffening means 24 passes axially through the electrode cable device 2, and the stiffening means 24 accordingly supports both the electrical conductors 8 and 16. In the corresponding manner, the stiffening means 26 supports both the insulated electrical conductors 10 and 18. The stiffening means 28 and 30 corrspondingly support the electrical conductors 12 and 20 and 14 and 22 respectively.

The proximal ends of the pre-shaped stiffening means 24-30 are connected to one end of a joint electrode cable connector 32 on the proximal end 4 of the electrode cable device 2. The electrode cable connector 32 is for connecting the electrode cable device 2 to a heart stimulator or pacemaker (not shown). At the lower end, as shown in FIG. 1, the connector 32 is equipped with a stylet orifice 34 through which a stylet 26, shown in FIG. 2, can be inserted into a channel means 38 in the electrode cable device 2. The channel means 38 extends axially along the length of the cable device from the connector 32 to the electrode contact means 41 at the distal end 6 of the cable device 2. The channel means 38 can be made of e.g. a thin, relatively stiff but flexible tube into whose longitudinal channel 40 the stylet 36 can be introduced in order to achieve axial stretching of the electrode cable device 2, from the state shown in FIG. 1 to an essentially straight state shown in FIG. 2, making it much easier to introduce the electrode cable device (preferably via a vein) into the heart. The stylet 36 is manipulated during its insertion in and out of the channel means 38 with fixed handle 42 at the proximal end of the stylet 36.

In this instance, each of the cable device's 2 thin, insulated electrical conductors 8-22 is connected to an associated implantable electrode contact means 44, 46, 48, 50, 52, 54, 56, 58 electrically connected to the conductor. The electrode contact means 44-58, devised as microelectrodes, are intended to be brought into permanent electrical contact with tissue in the wall of the cavity in a human body in which they have been implanted.

The electrode cable device 2 shown in FIGS. 1 and 2 is designed, preferably, for transmitting electrical signals between a heart stimulator, connected to the connector 32 , and the two cavities in a human heart in which the contact means 44-58 are to be implanted to achieve electrical contact. The four electrode contact means 52, 54, 56 and 58 are arrayed in a first contact area of the cable device, whereas the other four electrode contact means 44, 46, 48, 50 are arrayed in a second contact area, separate from the first contact area in the cable device's 2 longitudinal direction, which is closer to the cable device's 2 proximal end 4 than the first contact area (with contact means 52-58).

In the illustrated instance, the first contact area with the contact means 52-58 is intended for the left ventricle in a heart, whereas the second contact area with the contact means 44-50 is intended for the left atrium of a heart.

So the electrode contact means 44-50 and 52-58 respectively are intended to achieve permanent electrical contact with tissue in the wall of the cavity in which they are arranged. In order to achieve this desired permanent electrical contact with the wall of the cavity in question, the electrode contact means are kept, at an initial stage of their implantation, pressed against the wall of the cavity with tension exerted by pre-shaped stiffening means 24, 26, 28, 30, running along the conductors, which support the conductors 8-22. These four elongate stiffening means 24-50, connected to and running along the conductors, are made of an inherently elastic material which is resorbable *in vivo.* The stiffening means 24-30 are pre-shaped in such a way that they impart an arched shape to their respective conductors, at least in the parts of the conductors in which the contact means 44-58 are located. Both the stiffening means 24-30 and the electrical conductors 8-22 supported by them are arranged in pairs so they lie on a common plane, the conductors in each such conductor pair arching outward in opposite directions. This circumstance is not very apparent in FIG. 1 but rather clearly depicted in the sectional views in FIGS. 3 and 4, showing the A-A and B-B section lines in FIG. 1.

The electrode cable device 2 shown in FIG. 1 therefore contains four pair of insulated electrical conductors, viz. pairs 8-12, 10-14, 16-20 and 18-22. The conductor pairs 16-20 and 18-20 have their electrode contact means 52-58 located in the device's first contact area, in which section A-A is located, whereas the other two conductor pairs 8-12 and 10-14 have their electrode contact means 44-50 located in the device's second contact area, in which section B-B is located.

The electrical conductors 8-22 with their associated stiffening means 24-30 made of resorbable means and the channel means 38 for the stylet 36 are bundled into a single string in a first cable section L1, located between the proximal end 4 of the device, and the second contact area (containing the B-B section), located between the device's two axially separate contact areas.

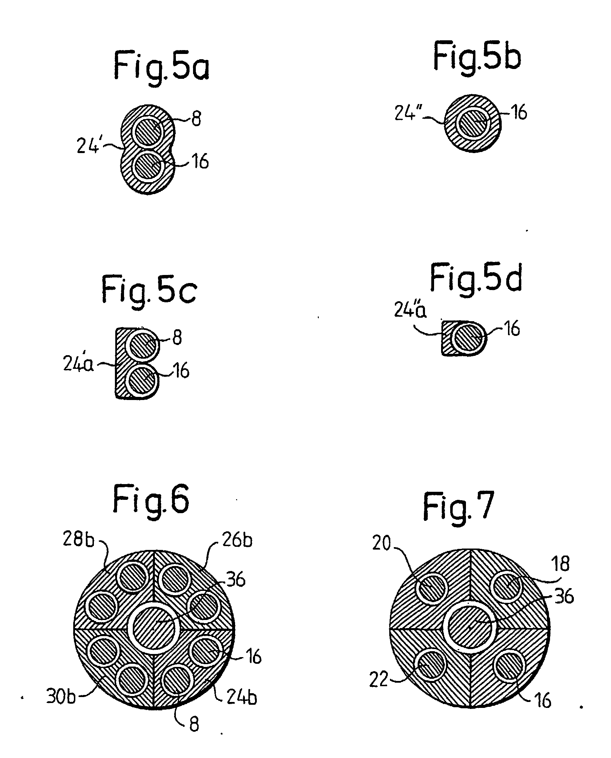

The stiffening means, such as the means 24, for each insulated electrical conductor, such as conductor 8 and 16, consists of a sleeve, enclosing the conductor, made of resorbable material, said sleeve being casing-like or tubular. At section C-C in FIG. 1, the two conductors 8 and 16 run parallel to each other, the stiffening means 24' being sleeve-like and displaying the 8-shaped cross-section shown in FIG. 5a. In FIG. 5b, which shows the section D-D, the stiffening means 24" can suitably have a circular, tubular shape, since only a single conductor 16 is involved, since the conductor 8 only extends to the electrode contact means 44.

As an alternative to tubular or sleeve-shaped means, the stiffening means 24, 26, 28 and 30 for each insulated electrical conductor could consist of a band-shaped or wire-shaped stiffening means running along and attached to the conductor. FIGS. 5c and 5d show examples of such a band-shaped stiffening means 24a' and 24a", said stiffening means being broader at section C-C shown in FIG. 5c than at section D-D shown in FIG. 5d.

FIG. 6 shows a conceivable cross-section E-E through the posterior cable section L1, bundled into a single cable string, in FIG. 1. Here, the stiffening means 24b for e.g. the conductors 8 and 16, are devised as a string-like means with an approximately quarter circle-shaped cross-section, the three other stiffening means 26b, 28b and 30b displaying a corresponding quarter circle-shaped cross-section, so the four string-shaped means 24b, 26b, 28b and 30b jointly give the cable device a circular cross-section for the posterior cable section L1.

The channel means 38 for the stylet 36 then consists of a longitudinal channel 40 inside the cable section L1 with a cross-section which, in total, is circular.

FIG. 7 shows a corresponding cross-section F-F through the anterior channel section L2. Since this channel section only contains the conductors 16, 18, 20 and 22 for the electrode contact means 52, 54, 56 and 58, the string-shaped channel section L2 acquires the cross-sectional shape shown in FIG. 7 with only one conductor in each quarter circle string.

As shown at the top of FIG. 1, the distal ends of the conductors 16, 18, 20, 22 in the anterior, first contact area are connected to the channel means 38 located on the distal end 60 of the cable device 2.

## Claims

1. An electrode cable device (2), which has a proximal end (4) and a distal end (6) and which comprises one or a plurality of thin, insulated electrical conductors (8-22) with a proximal end and a distal end, the conductor, or at least one of the conductors, being equipped with at least one implantable electrode contact means (44-58), in electrical contact with the conductor, to be brought into permanent electrical contact with tissue in the wall of a cavity in a human body, such as the wall of the atrium or ventricle in the heart, said device being devised so the respective contact means (44-58) can be kept pressed, as the result of pre-tensioning, against the cavity wall, the conductor, or the conductors (8-22), in the electrode cable device (2) being supported by an elongate, pre-shaped stiffening means (24,26,28,30), connected to and running along the conductor, made of an inherently elastic material , said stiffening means impart an arching or helical shape to at least a section of the conductor in the area in which its contact means (44-58) are located, **characterized in that**, said elastic material is resorbable in vivo, and the electrode cable device also includes a longitudinal channel means (38) into which a stylet (36) can be inserted for temporary, at least essentially linear stretching of the cable device and its conductor's arching or helical section.

2. A cable device according to claim 1, the device comprising at least one pair, preferably two pairs, of thin, insulated electrical conductors, **characterized in that** each conductor (8-22) in each conductor pair carries a electrode contact means, devised as a microelectrode (44-58) arranged on a convex, curving section of the conductor arching out from the cable device's center line, each conductor in each conductor pair bulging outward in opposite directions.

3. A cable device according to claim 1 or 2, **characterized in that** the device includes two or a plurality of pairs of insulated electrical conductors (8-22), at least a first conductor pair having their associated electrode contact means (52-58) arranged in a first contact area of the cable device, and at least a second conductor pair having their associated electrode contact means (44-50) arranged in a second contact area, longitudinally separate from the first contact area, the second contact area being closer to the device's proximal end (4) than the first contact area.

4. A cable device according to claim 3, **characterized in that** the device has four pairs of insulated electrical conductors (8-22), two of the conductor pairs having their electrode contact means (52-58) located in the device's first contact area, the other two conductor pair having their electrode contact means (44-50) in the device's second contact area.

5. A cable device according to claim 3 or 4, **characterized in that** the electrical conductors (8-22), their associated stiffening means (24-30) made of resorbable material and the channel means (38) for a stylet (36) are bundled into a single cable string in a first cable section (L1), located between the proximal end (4) of the device and the second contact area, and a second, anterior cable section (L2), located between the first and the second contact area.

6. A cable device according to any of the previous claims, **characterized in that** the stiffening means (24,26,28,30) for each insulated electrical conductor consists of a tubular sleeve (24',26',28',30',24'',26'',28'',30'') enclosing the conductor.

7. A cable device according to any of claims 1-5, **characterized in that** the stiffening means (24,26,28,30) for each insulated electrical conductor consists of band-shaped or wire-like stiffening means (24a',24a'';26a',26a'', etc.) which runs along and is attached to same.

8. A cable device according to claim 5, **characterized in that** the stiffening means for the conductors in each conductor pair consists of a string-shaped means (24b,26b,28b,30b), which wholly or partially encloses both conductors, the string-shaped means for the conductor pairs in the cable device being devised in the cable device's bundled cable strings, so the total cross-section of the cable device is circular at these strings.

9. A cable device according to claim 8, **characterized in that** the channel means (38) for a stylet (36) consists of a longitudinal channel inside the cable device's cable string sections with a round cross-section.

10. A cable device of any of claims 3-9, **characterized in that** the distal ends of the conductors (16-22) in the anterior, first contact area are connected to the end (60) of the channel means located on distal end (6) of the cable device (2).

11. A cable device of any of the previous claims, **characterized in that** the stiffening means (24,26,28,30) is made of a biodegradable polymer material which can be made to assume an elastic, resilient state.

## Patentansprüche

1. Elektrodenkabelvorrichtung (2), die ein proximales Ende (4) und ein distales Ende (6) aufweist und einen oder mehrere dünne, isolierte elektrische Leiter (8-22) mit einem proximalen Ende und einem distalen Ende enthält, wobei der Leiter oder wenigstens einer der Leiter mit wenigstens einem implantierbaren Elektrodenkontaktmittel (44-58), das mit dem Leiter in elektrischer Verbindung steht, ausgerüstet ist, um mit Gewebe in der Wand eines Hohlraums in einem menschlichen Körper, wie der Wand des Atriums oder Ventrikels im Herzen, in permanenten elektrischen Kontakt gebracht zu werden, die genannte Vorrichtung so ausgelegt ist, dass das betreffende Kontaktmittel (44-58) als Folge eines Vorspannens gegen die Hohlraumwand gedrückt gehalten werden kann, der Leiter oder die Leiter (8-22) in der Elektrodenkabelvorrichtung (2) gestützt wird/werden durch ein längliches, vorgeformtes Versteifungsmittel (24, 26, 28, 30), das mit dem Leiter verbunden ist und längs des selben läuft, und das aus einem inhärenten, elastischen Material besteht, das genannte Versteifungsmittel wenigstens einem Abschnitt des Leiters in dem Bereich, in dem seine Kontaktmittel (44-58) gelegen sind, eine gewölbte oder gewundene Form verleiht, **dadurch gekennzeichnet, dass** das genannte elastische Material in vivo resorbierbar ist und die Elektrodenkabelvorrichtung auch eine längliche Kanalvorrichtung (38) enthält, in die ein Mandrin (36) einsetzbar ist, für ein temporäres, wenigstens im wesentlichen lineares Strecken der Kabelvorrichtung und ihres gewölbten bzw. gewundenen Leiterabschnittes.

2. Kabelvorrichtung nach Anspruch 1 , wobei die Vorrichtung wenigstens ein Paar, vorzugsweise zwei Paare dünner isolierter elektrischer Leiter enthält, **dadurch gekennzeichnet, dass** jeder Leiter (8-22) in jedem Leiterpaar ein Elektrodenkontaktmittel trägt, das ausgelegt ist als eine Mikroelektrode (44-58), die auf einem konvexen, gekrümmten Abschnitt des Leiters angeordnet ist, der sich von der Mittellinie der Kabelvorrichtung auswölbt, wobei sich die Leiter in jedem Leiterpaar jeweils in entgegengesetzte Richtungen auswölben.

3. Kabelvorrichtung nach Anspruch 1 oder 2, **dadurch gekennzeichnet, dass** die Vorrichtung zwei oder eine Mehrzahl von Paaren von isolierten elektrischen Leitern (8-22) enthält, wenigstens ein erstes Leiterpaar seine zugehörigen Elektrodenkontaktmittel (52-58) in einem ersten Kontaktbereich der Kabelvorrichtung angeordnet hat und wenigstens ein zweites Leiterpaar seine Elektrodenkontaktmittel (44-50) in einem zweiten Kontaktbereich angeordnet hat, der in Längsrichtung vom ersten Kontaktbereich getrennt ist, wobei der zweite Kontaktbereich näher am proximalen Ende (4) der Vorrichtung als der erste Kontaktbereich liegt.

4. Kabelvorrichtung nach Anspruch 3, **gekennzeichnet, dass** die Vorrichtung vier Paare isolierter elektrischer Leiter (8-22) aufweist, wobei zwei der Leiterpaare mit ihren Elektrodenkontaktmitteln (52-58) im ersten Kontaktbereich der Vorrichtung gelegen sind, und die beiden anderen Leiterpaare ihre Elektrodenkontaktmittel (44-50) im zweiten Kontaktbereich der Vorrichtung haben.

5. Kabelvorrichtung nach Anspruch 3 oder 4, **dadurch gekennzeichnet, dass** die elektrischen Leiter (8-22), ihre zugehörigen Versteifungsmittel (24-30) aus resorbierbarem Material und die Kanalvorrichtungen (38) für einen Mandrin (36) in einem einzigen Kabelstrang in Form eines ersten Kabelabschnittes (L1) gebündelt sind, der zwischen dem proximalen Ende (4) der Vorrichtung und dem zweiten Kontaktbereich gelegen ist und ein zweiter, vorderer Kabelabschnitt (L2) zwischen dem ersten und dem zweiten Kontaktbereich gelegen ist.

6. Kabelvorrichtung nach einem der vorhergehenden Ansprüche, **dadurch gekennzeichnet, dass** das Versteifungsmittel (24, 26, 28, 30) für jeden isolierten elektrischen Leiter aus einer rohrförmigen Hülse (24', 26', 28', 30', 24", 26", 28", 30") gebildet ist, die den Leiter umschließt.

7. Kabelvorrichtung nach einem der Ansprüche 1 bis 5, **dadurch gekennzeichnet, dass** das Versteifungsmittel (24, 26, 28, 30) für jeden elektrischen Leiter aus einem bandförmigen oder drahtförmigen Versteifungsmittel (24a', 24a"; 26a', 26a"; etc) gebildet ist, das längs des Leiters verläuft und an diesem angebracht ist.

8. Kabelvorrichtung nach Anspruch 5, **dadurch gekennzeichnet, dass** das Versteifungsmittel für die Leiter in jedem Leiterpaar aus einem strangförmigen Mittel (24b, 26b, 28b, 30b) gebildet ist, das beide Leiter ganz oder teilweise umschließt, wobei das strangförmige Mittel für die Leiterpaare in der Kabelvorrichtung innerhalb der gebündelten Kabelstränge der Kabelvorrichtung so ausgebildet ist, dass der Gesamtquerschnitt der Kabelvorrichtung bei diesen Strängen kreisförmig ist.

9. Kabelvorrichtung nach Anspruch 8, **dadurch gekennzeichnet, dass** die Kanalvorrichtung (38) für einen Mandrin (36) innerhalb der Kabelstrangabschnitte der Kabelvorrichtung einen Längskanal mit einem runden Querschnitt umfasst.

10. Kabelvorrichtung nach einem den Ansprüche 3 bis 9, **dadurch gekennzeichnet, dass** die distalen Enden der Leiter (16-22) in dem vorderen, ersten Kontaktbereich mit dem Ende (60) der Kanalvorrichtung verbunden sind, die am distalen Ende (6) der Kabelvorrichtung (2) gelegen ist.

11. Kabelvorrichtung nach einem der vorhergehenden Ansprüche, **dadurch gekennzeichnet, dass** das Versteifungsmittel (24, 26, 28, 30) aus einem biologisch abbaubaren polymeren Material gebildet ist, das so hergestellt werden kann, dass es einen elastischen, federnden Zustand annimmt.

## Revendications

1. Dispositif (2) à câble d'électrode, qui a une extrémité (4) proximale et une extrémité (6) distale, et qui comporte un ou plusieurs conducteurs (8-22) minces électriquement isolés ayant une extrémité proximale et une extrémité distale, le conducteur, ou au moins l'un des conducteurs, étant muni d'au moins un moyen (44-58) à contact d'électrode implantable, en contact électrique avec le conducteur, pour être amené en contact électrique permanent avec du tissu dans la paroi d'une cavité d'un corps humain, tel que la paroi de l'oreillette ou du ventricule dans le coeur, le dispositif étant conçu de sorte que les moyens (44-58) à contact respectif peuvent être maintenus pressés, en résultat de la mise sous pré-tension, contre la paroi de cavité, le conducteur ou les conducteurs (8-22), dans le dispositif (2) à câble d'électrode, étant supporté par un moyen (24, 26, 28, 30) de raidissement oblong préformé connecté au conducteur et s'étendant le long de celui-ci, réalisé en un matériau de manière inhérente élastique, les moyens de raidissement impartissant une forme hélicoïdale ou en forme d'arche à au moins une section du conducteur dans l'aire dans laquelle ses moyens (54-58) à contact se trouvent, **caractérisé en ce que** le matériau élastique peut être résorbé in vivo et le dispositif à câble d'électrode comporte également des moyens (38) formant canal longitudinal dans lequel un stylet (36) peut être inséré pour un étirement temporaire au moins sensiblement linéaire du dispositif à câble et de sa section de conducteur hélicoïdale ou en forme d'arche.

2. Dispositif à câble suivant la revendication 1, le dispositif comportant au moins une paire, de préférence deux paires, de conducteurs minces électriquement isolés, **caractérisé en ce que** chaque conducteur (8-22) dans chaque paire de conducteurs porte des moyens à contact d'électrode, conçu en tant qu'une microélectrode (44-58) disposée sur une section incurvée convexe du conducteur formant un arche vers l'extérieur à partir de la ligne centrale du dispositif à câble, chaque conducteur dans chaque paire de conducteurs étant bombé vers l'extérieur dans des directions opposées.

3. Dispositif à câble suivant la revendication 1 ou 2, **caractérisé en ce que** le dispositif comporte deux paires ou une pluralité de paires de conducteurs (8-22) électriques isolées, au moins une première paire de conducteurs ayant leurs moyens (52-58) à contact d'électrode associés disposés dans une première aire de contact du dispositif à câble, et au moins une deuxième paire de conducteurs ayant leurs moyens (44-50) à contact d'électrode associés disposés dans une deuxième aire de contact, longitudinalement distincte de la première aire de contact, la deuxième aire de contact étant plus proche de l'extrémité (4) proximale du dispositif que ne l'est la première aire de contact.

4. Dispositif à câble suivant la revendication 3, **caractérisé en ce que** le dispositif comporte quatre paires de conducteurs (8-22) électriquement isolés, deux des paires de conducteurs ayant leurs moyens (52-58) à contact d'électrode situés dans la première aire de contact du dispositif, les deux autres paires de conducteurs ayant leurs moyens (44-50) à contact d'électrode dans la deuxième aire de contact du dispositif.

5. Dispositif à câble suivant la revendication 3 ou 4, **caractérisé en ce que** les conducteurs (8-22) électriques, leurs moyens (24-30) de raidissement associés réalisés en matériau résorbable et les moyens (38) formant canal pour un stylet (36) sont formés en faisceau en une chaîne à câble unique dans une première section (L1) de câble, située entre l'extrémité (4) proximale du dispositif et la deuxième aire de contact, et une deuxième section (L2) à câble antérieure, située entre la première aire de contact et la deuxième aire de contact.

6. Dispositif à câble suivant l'une quelconque des revendications précédentes, **caractérisé en ce que** les moyens (24, 26, 28, 30) de raidissement pour chaque conducteur électrique isolé sont constitués d'un manchon (24', 26', 28', 30', 24", 26", 28", 30") tubulaire enfermant le conducteur.

7. Dispositif à câble suivant l'une quelconque des revendications 1 à 5, **caractérisé en ce que** les moyens (24, 26, 28, 30) de raidissement pour chaque conducteur électrique isolé sont constitués de moyens (24a', 24a" ; 26a', 26a", etc.) de raidissement en forme de fil ou en forme de bande qui s'étendent le long du conducteur et sont fixés à celui-ci.

8. Dispositif à câble suivant la revendication 5, **caractérisé en ce que** le moyen de raidissement pour les conducteurs dans chaque paire de conducteurs est constitué d'un moyen (24b, 26b, 28b, 30b) en forme de chaîne, qui enferme partiellement ou complètement les deux conducteurs, les moyens en forme de chaîne pour les paires de conducteurs dans le dispositif à câble étant conçus dans les chaînes de câble en faisceau du dispositif à câble, de sorte que la section transversale totale du dispositif à câble est circulaire au niveau de ces chaînes.

9. Dispositif à câble suivant la revendication 8, **caractérisé en ce que** les moyens (38) formant canal pour un stylet (36) sont constitués d'un canal longitudinal à l'intérieur des sections à chaîne de câble du dispositif à câble avec une section transversale arrondie.

10. Dispositif à câble suivant l'une quelconque des revendications 3 à 9, **caractérisé en ce que** les extrémités distales des conducteurs (16-22) dans la première aire de contact antérieure sont connectées à l'extrémité (60) des moyens formant canal situés sur l'extrémité (6) distale du dispositif (2) à câble.

11. Dispositif à câble suivant l'une quelconque des revendications précédentes, **caractérisé en ce que** les moyens (24, 26, 28, 30) de raidissement sont réalisés en un matériau polymère biodégradable qui peut être réalisé de manière à prendre un état élastique résilient.
